# EUROPEAN PATENT SPECIFICATION

(11) **EP 4 327 846 B1**
(45) Date of publication and mention of the grant of the patent: **17.12.2025**
(21) Application number: 22191453.4
(22) Date of filing: 22.08.2022
(51) Int. Cl.: A61M 5/14, A61M 5/50

(54) **DRIP CHAMBER DEVICE FOR USE ON AN INFUSION LINE FOR ADMINISTERING A MEDICAL FLUID FROM A MEDICAL CONTAINER TO A PATIENT**
TROPFKAMMERVORRICHTUNG ZUR VERWENDUNG AUF EINER INFUSIONSLEITUNG ZUR VERABREICHUNG EINER MEDIZINISCHEN FLÜSSIGKEIT AUS EINEM MEDIZINISCHEN BEHÄLTER AN EINEN PATIENTEN
DISPOSITIF DE CHAMBRE DE GOUTTE-À-GOUTTE DESTINÉ À ÊTRE UTILISÉ SUR UNE LIGNE DE PERFUSION POUR ADMINISTRER UN FLUIDE MÉDICAL DEPUIS UN RÉCIPIENT MÉDICAL À UN PATIENT

(43) Date of publication of application: 28.02.2024
(73) Proprietor: Fresenius Kabi Deutschland GmbH, 61352 Bad Homburg (DE)
(72) Inventor: Baumgart, Steffen, 36088 Hünfeld (DE)
(74) Representative: Fresenius Kabi Deutschland GmbH

(56) References cited:
- WO-A1-2022/233486
- US-A- 3 311 268
- US-A1- 2007 257 065

## Description

The instant invention relates to a drip chamber device for use on an infusion line for administering a medical fluid from a medical container to a patient.

A drip chamber device of this kind comprises a chamber body forming a wall confining an inner space of the chamber body, an inflow end for allowing the medical fluid to flow into said inner space of the chamber body in a downstream direction, and an outflow end for allowing the medical fluid to exit from said inner space of the chamber body in said downstream direction.

A drip chamber device of this kind typically is used on an infusion line to allow gas, such as air, to rise out from a fluid so that it is not passed downstream towards a patient. A drip chamber device is typically employed in delivery systems for intravenous therapy and acts to prevent air embolism. The drip chamber device herein at its inflow end is connected to a medical container such that a medical fluid may enter into the drip chamber device from the medical container. A tubing of an infusion line set is connected to the drip chamber device at its outflow end such that the medical fluid may flow into the tubing from the drip chamber device, a fluid flow being caused and driven by gravity. The drip chamber device herein also serves to allow a user to estimate the rate at which fluid is administered to the patient.

When using a drip chamber in connection with a tubing of an infusion line, the drip chamber and the tubing need to be primed in that the tubing downstream of the drip chamber is filled with fluid prior to connecting the tubing to an appropriate access port for delivering the fluid towards a patient. In particular, gas, such as air, needs to be removed from the tubing such that air embolism is prevented. For this, in nowadays conventional drip chambers the chamber body of the drip chamber device is flexible such that it may be squeezed in order to manually pump fluid from the container connected to the drip chamber device at the inflow end into the tubing of the infusion line connected to the drip chamber device at the outflow end. As the squeezing needs to be repeated multiple times to fill the chamber body and the connected tubing with fluid, the priming may be cumbersome for a user and may require a significant time.

US 2007/257065 A1 discloses a fluid dispensing device which ensures fluid flow even when the reservoir is held too low such that fluid cannot flow due to gravity.

It is an object of the instant invention to provide a drip chamber device, an infusion line set and a method for priming a drip chamber device which allow in an easy and user-friendly manner to cause fluid to enter into the drip chamber device for priming the drip chamber device and the tubing of an infusion line connected to the drip chamber device.

This object is achieved by means of a drip chamber device comprising the features of claim 1.

Accordingly, the drip chamber device comprises an adjustment member which is movable relative to the wall of the chamber body. The adjustment member comprises a closing section which in a first position of the adjustment member defines a first volume of the inner space and is movable within the chamber body to change the volume of the inner space. The drip chamber device comprises an adjustment member which acts as a piston in that it is movable in the chamber body of the drip chamber device to alter the volume of the inner space confined within the chamber body. The inner space herein is defined by the chamber body together with the closing section of the adjustment member, such that by moving the closing section of the adjustment member within the chamber body the volume of the inner space is changed by moving the adjustment member with respect to the chamber body.

In particular, the adjustment member is movable within the chamber body to increase the volume of the inner space in comparison to the first volume which the inner space assumes in the first position of the adjustment member with respect to the chamber body. In an initial position the adjustment member may be in the first position with respect to the chamber body, such that the closing section together with the chamber body defines an inner space having a first volume, which is comparatively small. The first volume beneficially is non-zero, such that even in the first position of the adjustment member it may be observed whether drips fall into the chamber body by fluid entering from the container. By moving the adjustment member with respect to the chamber body to increase the volume of the inner space, fluid may be caused to enter into the chamber body in a syringe-like pumping action by means of an under pressure caused within the chamber body as a result of the movement of the adjustment member. For priming the drip chamber device and a tubing of an infusion line connected to the outflow end of the drip chamber device, fluid hence is caused to enter into the chamber body by movement of the adjustment member such that the drip chamber device is at least partially filled with fluid.

The closing section is movable within the chamber body. Beneficially, herein, the closing section is slidably arranged to be moved along the wall of the chamber body, such that a transition in between the closing section and the wall of the chamber body is sealed in a fluid-tight manner. The closing section hence, similar to a piston of a syringe, acts to define the volume of the inner space within the chamber body, wherein the volume is variable by movement of the adjustment member.

In one embodiment, the inflow end is arranged on the chamber body. Through the inflow end fluid may enter into the chamber body, wherein the chamber body at the inflow end is connected to the container. For example, at the inflow end a spike element may be arranged by which a closure element, for example a membrane, of the container may be pierced in order to connect the drip chamber device to the container. When the drip chamber device is connected to the container, fluid may flow from the container through the inflow end into the chamber body of the drip chamber device, wherein the flow connection is such that drips are formed which due to gravity fall into the chamber body.

The adjustment member comprises a line section connected to the closing section, the line section forming the outflow end of the drip chamber device. The line section serves to conduct fluid from the inner space confined by the chamber body, such that fluid may exit from the chamber body via the line section of the adjustment member. The line section of the adjustment member is movable together with the closing section when moving the adjustment member with respect to the chamber body. To the line section, for example, a tubing of an infusion line may be connected such that fluid may enter into the tubing via the line section.

The line section may for example be rigidly formed on the closing section and may protrude from the closing section along a longitudinal direction.

The adjustment member comprises a stopper member configured to allow a first actuation movement of the adjustment member towards a second position in a first direction for increasing the volume of the inner space, but to stop a second actuation movement of the adjustment member from the second position in a second direction opposite to the first direction.

The adjustment member is movable with respect to the chamber body of the drip chamber device from the first position. By moving the adjustment member from the first position towards the second position, the volume of the inner space within the chamber body is increased such that fluid is sucked into the chamber body. When the second position is reached the inner space assumes a maximum volume, wherein the chamber body of the drip chamber device is filled (at least) by an amount of fluid equal to the volume increase caused by the movement of the adjustment member from the first position.

Whereas a movement of the adjustment member from the first position towards the second position is not hindered by the stopper member, a second actuation movement counter to the first actuation movement is stopped after a predefined distance by the stopper member. By means of the second actuation movement the volume of the inner space is reduced, wherein the amount of volume decrease is defined by the distance of movement in the second direction before the movement is stopped by the stopper member. By means of the second actuation movement fluid is forced from the chamber body into a tubing connected to the line section of the adjustment member, such that during the second actuation movement the tubing is primed and filled with fluid.

Accordingly, the stopper member is configured to stop the second actuation movement after a decrease of the volume of the inner space by a predefined volume with respect to the volume of the inner space in the second position. The stopper member in particular is configured to allow a certain movement of the adjustment member in the second direction from the second position, wherein after a certain, predefined distance the stopper member stops a further movement. By means of the second actuation movement, hence, the inner space is reduced by a predefined volume, such that fluid is forced to exit from the chamber body and to flow via the outflow end into a tubing connected to the drip chamber device for priming the tubing.

A priming of the drip chamber device and a tubing connected to the drip chamber device hence is possible by performing two actuation movements. In a first actuation movement the adjustment member is moved with respect to the chamber body of the drip chamber device in order to increase the volume of the inner space within the chamber body, such that fluid is sucked into the chamber body from a container connected to the drip chamber device. In a second actuation movement, then, a certain amount of fluid is forced to flow out of the chamber body into a connected tubing such that the tubing is filled with fluid.

In one embodiment, the stopper member comprises a stop element adjustable with respect to the closing section of the adjustment member between a release configuration and a stop configuration, wherein the stop element is configured to assume the release configuration at least in the beginning of the first actuation movement of the adjustment member and to assume the stop configuration in the second position of the adjustment member to stop the second actuation movement of the adjustment member. The stop element may for example be arranged on the line section connected to the closing section and may be adjustable with respect to the line section.

Herein, a pre-tensioning element may pre-tension the stop element towards the stop configuration, the pre-tensioning element being shaped for example by a mechanical spring element or another elastic pre-tensioning device.

In one embodiment, the stop element in the first position of the adjustment member is held in the release configuration by interaction with the chamber body. For example, in the first position of the adjustment member the stop element may be received within the chamber body such that the stop element is held in the release configuration. By moving the adjustment member with respect to the chamber body for increasing the volume of the inner space confined within the chamber body the stop element is caused to exit from the chamber body, wherein the stop element is transferred into its stop configuration for example by the pre-tensioning element once the stop element is no longer held in the release configuration by means of the chamber body.

In the release configuration the adjustment member may for example be radially confined by the chamber body. In the stop configuration, in turn, the stop element for example is radially widened, such that a counter movement from the second position of the adjustment member in the second direction for reducing the volume of the inner space is stopped by interaction of the stop element with the chamber body.

In one embodiment, the chamber body comprises a floor in which an opening is formed. When moving the adjustment member in the first actuation movement towards a second position for increasing the volume of the inner space, the stop element is moved through the opening in the floor, such that the stop element is radially compressed until it has passed the opening. After having passed the opening the stop element is radially widened, such that a counter movement is stopped by interaction of the stop element with the chamber body.

If the drip chamber device shall be used anew, the stop element may for example be radially compressed by hand such that it can be moved through the opening in the floor.

In one embodiment, the stop element comprises a first detent device which interacts with a second detent device of the chamber body after the second actuation movement. After the stop element has performed the second actuation movement and hence after the volume of the inner space has been decreased by said predefined volume, the first detent device of the stop element comes to engage with the second detent device of the chamber body such that a detent connection between the stop element and the chamber body is established. The stop element hence is held in position with respect to the chamber body after the second actuation movement, such that the adjustment member may not be moved out of place without further ado in said first direction to again increase the volume of the inner space.

In one embodiment, the drip chamber device comprises a first valve device arranged at the inflow end. Additionally or alternatively, the drip chamber device comprises a second valve device arranged at the outflow end. At least one of the valve devices may for example be configured as a non-return valve, such that fluid may flow from the container into the chamber body of the drip chamber device through the inflow end and/or fluid may exit from the drip chamber device at the outflow end only in the downstream direction.

In one embodiment, at least one of the valve devices may act as an anti-siphon valve. For this, the corresponding valve device may be configured to allow fluid to pass in the downstream direction only if a fluid pressure at the valve device in the downstream direction is larger than a predefined threshold.

In particular, the second valve device at the outflow end of the drip chamber device may act as an anti-siphon valve. In this way it can be achieved that fluid may exit from the drip chamber device only if a sufficient fluid pressure, caused gravitationally by the amount of fluid in the chamber body of the drip chamber device and in the tubing, is present at the valve device. In this way a gravity flow from the drip chamber device may be prevented for example during the first actuation movement. In addition, it is prevented that air may flow into a tubing connected to the drip chamber device when a container connected to the drip chamber device is empty.

The threshold of the fluid pressure may for example correspond to a value equal to or larger than the fluid pressure caused by the static height of fluid within the chamber body in the second position of the adjustment member with respect to the chamber body.

In one embodiment, an infusion line set comprises a tubing for conducting a medical fluid and a drip chamber device of the kind described above connected to the tubing.

In another aspect, a method for priming a drip chamber device of the kind described above comprises: moving the adjustment member of the drip chamber device relative to the chamber body of the drip chamber device from the first position towards a second position to increase the volume of the inner space with respect to the volume of the inner space in the first position, and moving the adjustment member of the drip chamber device relative to the chamber body of the drip chamber device from the second position to decrease the volume of the inner space with respect to the volume of the inner space in the second position.

The advantages and advantageous embodiments described above for the drip chamber device equally apply also to the method.

During the method, the priming of the drip chamber device takes place by performing two actuation movements.

In the first actuation movement, the adjustment member is moved relative to the chamber body of the drip chamber device from its first, initial position towards a second position to increase the volume of the inner space confined in the chamber body. In the first position of the adjustment member the inner space assumes a small (minimum) volume, which however may be sufficiently large such that a user may observe whether drips are falling into the chamber body of the drip chamber device. During the first actuation movement, fluid is sucked from the container into the chamber body such that the inner space of the chamber body at least partially is filled with the medical fluid entering into the drip chamber device from the container. At the end of the first actuation movement, the adjustment member assumes a second position with respect to the chamber body, at which the volume of the inner space is at its maximum.

During a second actuation movement, then, the adjustment member is moved from the second position in a counter direction to now reduce the volume of the inner space, hence forcing fluid from the chamber body via the outflow end into a tubing connected to the determined device, for example to a line section of the adjustment member. Hence, during the second actuation movement at least a portion of the fluid in the chamber body is forced to flow into the tubing, such that the tubing is filled with medical fluid and hence is primed.

During the second actuation movement, the volume of the inner space beneficially is reduced by a predefined amount, corresponding for example to a distance by means of which the adjustment member is moved with respect to the chamber body in the second direction before a further movement is stopped by the stopper member. The tubing hence is filled by an amount of fluid corresponding to the volume reduction of the inner space. The design of the adjustment member herein may be adapted to the type of tubing connected to the drip chamber device, such that the tubing during the second actuation movement may beneficially be fully filled with fluid.

In that the priming may take place by two distinct actuation movements, the priming is easy and may be accomplished in a user-friendly manner. In particular, no repeated squeezing action at the drip chamber device is required, hence facilitating the use of the drip chamber device for a user.

The idea underlying the invention shall subsequently be described in more detail with respect to the embodiment shown in the drawings. Herein:
- Fig. 1: shows a drip chamber device used on an infusion line and connected to a medical container;

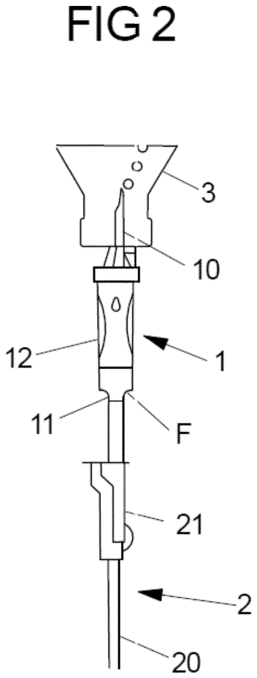

- Fig. 2: shows a drip chamber device during priming;
- Fig. 3: shows a drip chamber device and a connected tubing in a primed state;
- Fig. 4: shows an embodiment of a drip chamber device comprising a movable adjustment member;
- Fig. 5A: shows the drip chamber device of Fig. 4 in an initial state;
- Fig. 5B: shows the drip chamber device after a first actuation movement of the adjustment member;
- Fig. 5C: shows the drip chamber device after a second actuation movement of the adjustment member;
- Fig. 6A: shows a drip chamber device, in another embodiment, in an initial state;
- Fig. 6B: shows the drip chamber device during a first actuation movement for increasing a volume of a chamber body;
- Fig. 6C: shows the drip chamber device after the first actuation movement of the adjustment member;
- Fig. 6D: shows the drip chamber device after a second actuation movement of the adjustment member;
- Fig. 7A: shows a drip chamber device, according to another embodiment, in an initial state;
- Fig. 7B: shows the drip chamber device after a first actuation movement; and
- Fig. 7C: shows the drip chamber device after a second actuation movement.

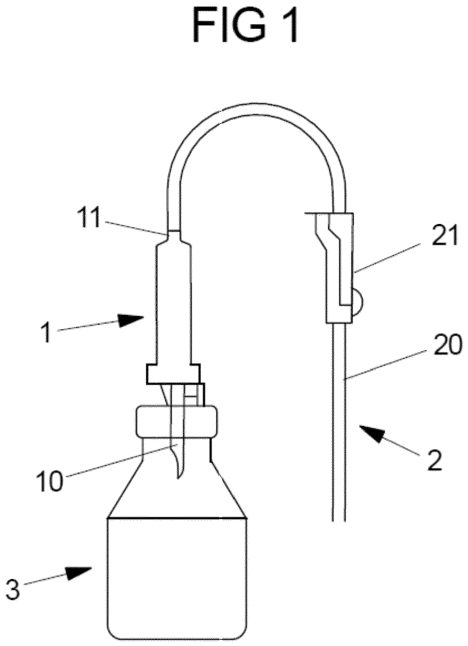

Fig. 1 shows a schematic drawing of a setup of a drip chamber device 1 connected to a tubing 20 of an infusion line 2 at an outflow end 11 and to a medical container 3 containing a medical fluid at an inflow end 10. At the inflow end 10, fluid may enter into the drip chamber device 1 from the medical container 3, wherein the access is such that drips fall into the drip chamber device 1. At the outflow end 11 fluid may exit from the drip chamber device 1 to flow in the tubing 20 towards a patient.

On the tubing 20, a control device 21 in the shape of a clamp is arranged. The control device 21 may be manually controlled in order to set a flow rate of fluid flowing through the tubing 20 and the drip chamber device 1 from the container 3.

Prior to employing a setup of a container 3 connected to an infusion line 2 as shown in Fig. 1 for performing an intravenous therapy on a patient, the drip chamber 1 and the tubing 20 connected to the drip chamber 1 need to be primed. For this, fluid is caused to enter into the drip chamber 1 in order to flow from the drip chamber 1 into the tubing 20 until the tubing 20 is filled with fluid.

For priming, in a first step the drip chamber device 1 is connected to the container 3 by piercing e.g. a membrane element of the container 3 by means of a spike forming the inflow end 10. Once the drip chamber device 1 is connected to the container 3, the container 3 for example is hung on a stand such that fluid may enter into the drip chamber device 1 from the container 3 by gravity.

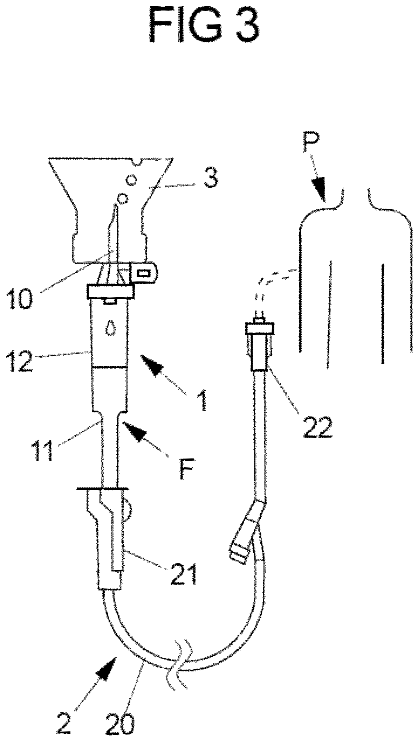

Herein, in order to cause a priming of the drip chamber device 1 and the tubing 20, a chamber body 12 of the drip chamber device 1 may, in a conventional drip chamber device 1, be squeezed by a user such that, by repeated squeezing, fluid is manually pumped from the container 3 into the drip chamber device 1 and is caused to flow into the tubing 20 until the tubing 20 is filled with fluid, as this is visible in Fig. 3.

Once the drip chamber device 1 and the tubing 20 of the infusion line 2 connected to the drip chamber device 1 is primed, the tubing 20 may be connected, using a connector 22, to an access port on a patient P such that the medical fluid of the container 3 is administered to the patient P in an intravenous infusion therapy.

By means of the drip chamber device 1 it is achieved that air may rise out from the fluid such that air embolism is prevented. In addition, by observing drips falling into the drip chamber a user may estimate a flow rate of the medical fluid through the infusion line 2 during administration. The flow through the tubing 20 herein is controlled by the control device 21, wherein drips fall into the drip chamber device 1 according to the flow resistance as set by the control device 21.

When using a conventional drip chamber 1, a repeated squeezing of the chamber body 12 is required in order to achieve a priming. This may be cumbersome and in addition requires a significant time to achieve a priming of the drip chamber device 1 and an infusion line 2 connected to the drip chamber device 1.

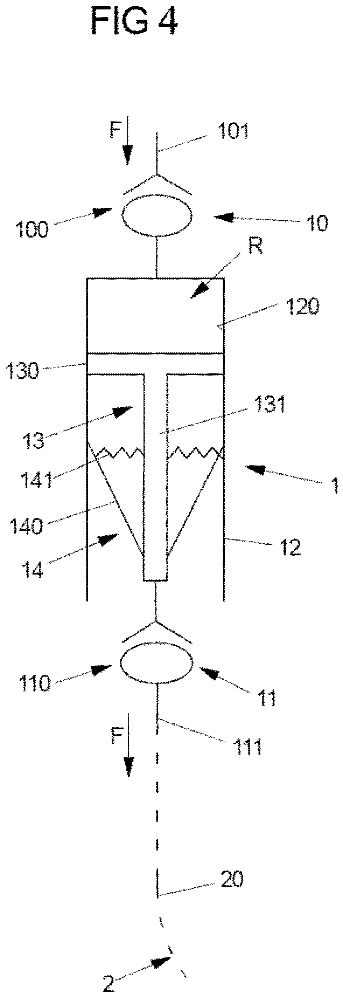

Referring now to Fig. 4, in order to ease a priming and in order to make a process for priming more comfortable and quicker for a user, it herein is proposed to use a movable adjustment member 13 on a drip chamber device 1, which allows to perform a pumping action on the drip chamber device 1 similar to the use of a piston of a syringe.

In the embodiment as shown in Fig. 4, a drip chamber device 1 comprises a chamber body 12 having for example a substantially cylindrical shape. The chamber body 12 forms a wall 120 circumferentially surrounding an inner space R within the chamber body 12.

An adjustment member 13 comprises a closing section 130 movable within the chamber body 12 by slidably moving the closing section 130 along the wall 120 of the chamber body 12, the closing section 130 together with the wall 120 defining the volume of the inner space R within the chamber body 12. A line section 131 is connected to the closing section 130, the line section 131 providing a conduit for fluid to exit from the inner space R of the chamber body 12.

An inflow end 10 is arranged on the chamber body 12 allowing fluid F to enter from a container 3 into the chamber body 12. A first valve device 100 configured as a non-return valve is arranged on a line section 101 at the inflow end 10 such that fluid may enter from the container 3 into the drip chamber device 1, but may not flow back into the container 3 from the drip chamber device 1.

An outflow end 11 is formed by the adjustment member 13. In particular, a line section 111 is connected to the line section 131 of the adjustment member 13, a second valve device 110 configured as a non-return valve being arranged on the line section 111. The line section may be part of the tubing 20 itself.

The adjustment member 13 comprises a stopper member 14 which is configured to allow a movement of the adjustment member 13 to increase the volume of the inner space R within the chamber body 12, but to stop (at least after a certain movement) a counter movement of the adjustment member 13 into the chamber body 12 for reducing the volume of the inner space R. The stopper member 14 comprises a stop element 140 and a pre-tensioning element 141. During a portion of a first actuation movement of the adjustment member 13 the stop element 140 is received and confined within the chamber body 12 such that the stop element 140 assumes a release configuration. Once the stop element 140 by movement of the adjustment member 13 is caused to exit from the chamber body 12, the stop element 140 assumes a stop configuration in that the stop element 140 is radially widened by means of the pre-tensioning element 141, such that a counter movement is stopped at a predefined position of the adjustment member 13 with respect to the chamber body 12.

By means of the adjustment member 13, an efficient priming of the drip chamber device 1 and a tubing 20 of an infusion line 2 connected to the drip chamber device 1 may be achieved.

For priming the drip chamber device 1, the drip chamber device 1 is connected to a medical container 3, similarly as it is shown in Fig. 1. The container 3 may then be hung on a post, such that fluid may flow from the container 3 by gravity into the drip chamber 1 and through the tubing 20 of the infusion line 2.

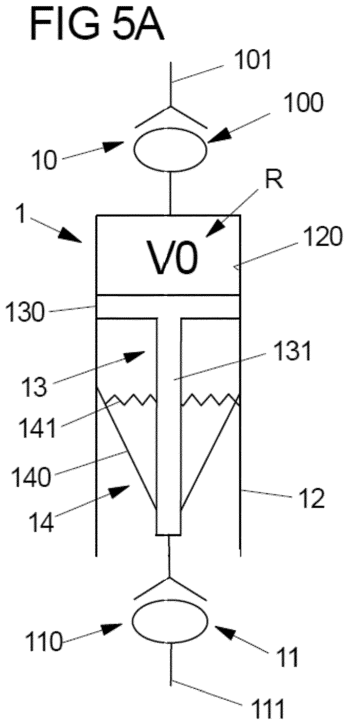

In an initial state the adjustment member 13 assumes a first position with respect to the chamber body 12, as it is shown in Fig. 5A. In the first position the inner space R assumes a volume V0 which is non-zero. The volume V0 in particular may be sufficiently large such that a user may observe whether drips are falling into the chamber body 12.

The first position for example may be defined by a stop on the chamber body 12 on which the adjustment member 13 rests in the first position.

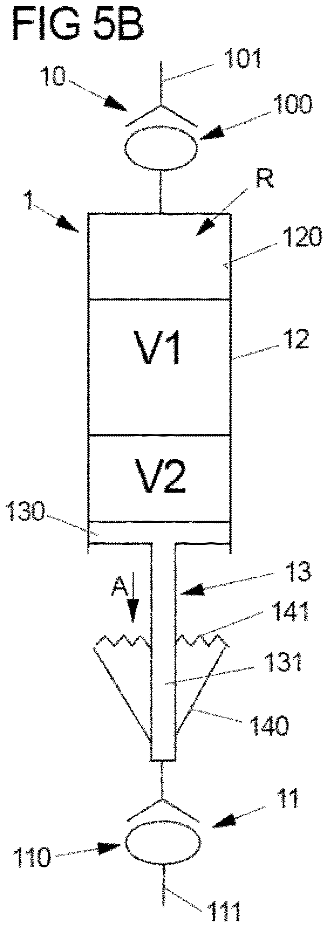

The priming then takes place by a first actuation movement A of the adjustment member 13 with respect to the chamber body to increase the volume of the inner space R, as it is shown in Fig. 5B. In particular, during the first actuation movement A the adjustment member 13 is moved from the first position of Fig. 5A into a second position as shown in Fig. 5B, in which the volume of the inner space R is increased by a volume amount V1+V2, as illustrated in Fig. 5B. Due to the first actuation movement A, liquid is sucked into the chamber body 12 of the drip chamber device 1 from the container 3 connected to the inflow end 10 of the drip chamber device 1, such that the inner space R is partially filled with a medical fluid corresponding to the increase volume V1+V2.

When moving the adjustment member 13 with respect to the chamber body 12 during the first actuation movement A, the stop element 140 of the stopper member 14 is caused to exit from the chamber body 12. Due to the pre-tensioning of the pre-tensioning element 141, configured for example as a mechanical spring element, the stop element 140 is radially widened, such that it may not be easily, at least not without a manual actuation, be reinserted into the chamber body 12.

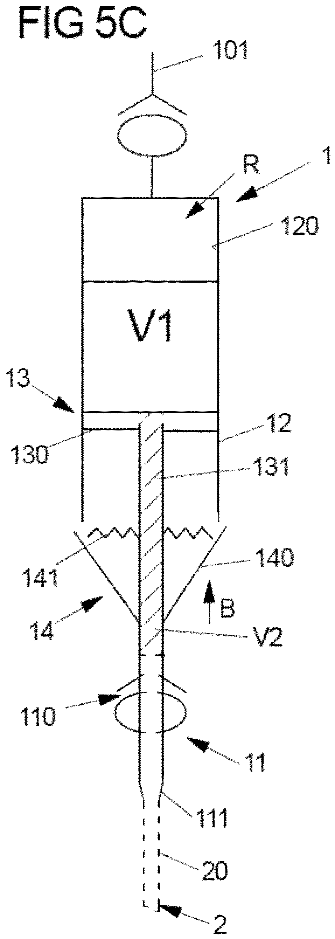

Following the first actuation movement A, priming is completed by a second actuation movement B in which the adjustment member 13 is moved into the chamber body 12 until a further movement is stopped by the stopper member 14, as illustrated in Fig. 5C. By means of the second actuation movement B the volume of the inner space R is reduced by a volume V2, such that an amount of fluid corresponding to the volume V2 is forced to exit from the chamber body 12 and to flow through the line section 131 and line section 111 into the connected tubing 20 of the infusion line 2. The tubing 20 hence is filled with fluid, such that at the end of the second actuation movement B air is removed from the tubing 20 and the infusion line 2 is primed.

The distance by which the adjustment member 13 is movable during the second actuation movement B may be designed in accordance with the type of infusion line 2 to be used on the drip chamber device 1. In particular, the volume V2 - by which the volume of the inner space R is reduced during the second actuation movement B - may be defined such that the tubing 20 of the infusion line 2 is fully filled with fluid during the second actuation movement B.

Once the second actuation movement B is completed, the drip chamber device 1 as well as the tubing 20 of the infusion line 2 connected thereto are primed. The infusion line 2 now may be connected to a patient P, such that intravenous therapy may start by delivering medical fluid from the container 3 through the drip chamber 1 and the infusion line 2 to the patient P.

The valve device 110 at the outflow end 11 may be configured as an anti-siphon valve which allows fluid to flow in the downstream direction towards the infusion line 2 only if a fluid pressure at the valve device 110 is larger than a predefined threshold. The threshold in particular may correspond to a fluid pressure caused by a static height of fluid of volume V1+V2 in the drip chamber 1 and the connected tubing 20.

By configuring the valve device 110 as an anti-siphon valve it may also be prevented that air may enter into the infusion line 2 if the container 3 is empty.

The stopper member 14 can be set back into the release configuration (corresponding to a non-actuated state of the adjustment member 13) to allow a new priming, for example in case of a replacement of the container 3. By moving the stop element 140 into its release configuration, the stopper member 14 may be reinserted into the chamber body 12 such that the adjustment member 13 may be moved into the first position (corresponding to the position of Fig. 5A).

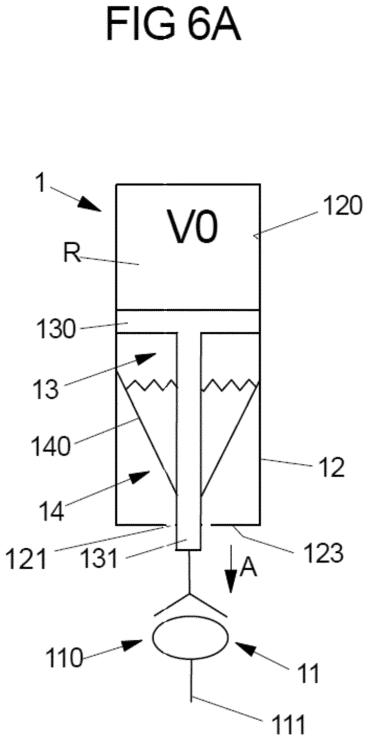

Referring now to Figs. 6A to 6D, in another embodiment a drip chamber device 1 comprises a chamber body 12 having a floor 123 in which an opening 121 is formed. An adjustment member 13 is movable within the chamber body 12 such that a closing section 130 is moved along an inner wall 120 of the chamber body. A line section 131 extends through the opening 121 within the floor 123 of the chamber body 120 and is connected, at an outflow end 11, to a tubing 20 (or may be formed by the tubing 20 itself).

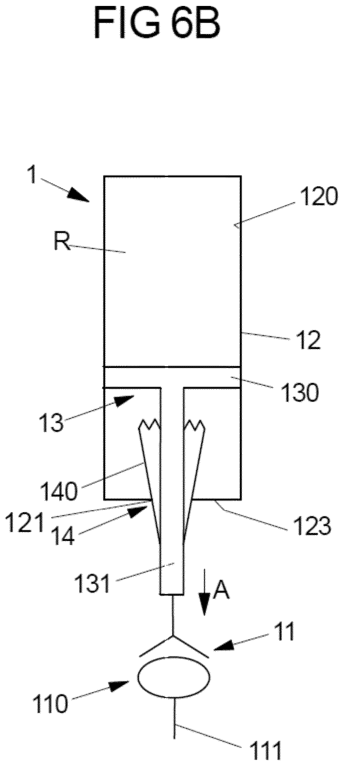

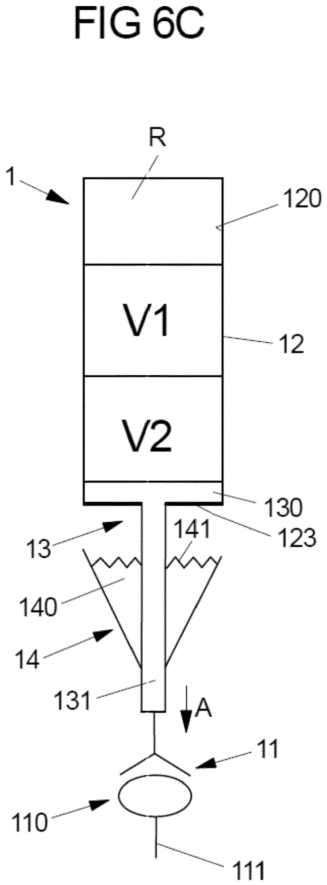

The embodiment of Figs. 6A to 6D differs from the embodiment as described above with respect to Figs. 4 and 5A to 5C in that a stopper member 14 with a stop element 140 during a first actuation movement A of the adjustment member 13 is moved through the opening 121 in the floor 123 such that the stop element 140, during the first actuation movement A, is radially compressed, as apparent from Figs. 6A and 6B. After having passed the opening 123 the stop element 140 is radially widened by action of a pre-tensioning element 141, such that the stop element 140 assumes the stop configuration according to Fig. 6C and, after a second actuation movement B counter to the first actuation movement A, interacts with the chamber body 12 to block a (further) movement.

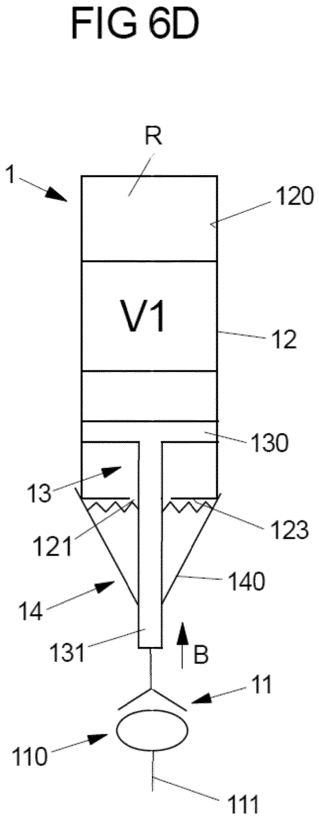

In one embodiment, the stop element 140 comprises a detent device 142 to interact with a corresponding detent device of the chamber body 12, such that in the position of Fig. 6D a detent connection between the stopper member 14 and the chamber body 12 is established. The adjustment member 13 hence is held in position after the second actuation movement B with respect to the chamber body 12, such that the adjustment member 13 may not without further ado be moved out of the primed position according to Fig. 6D.

Other than the noted differences, the embodiment of Figs. 6A to 6D is functionally identical to the embodiment of Figs. 4 and 5A to 5C, such that it also shall be referred to the explanations above.

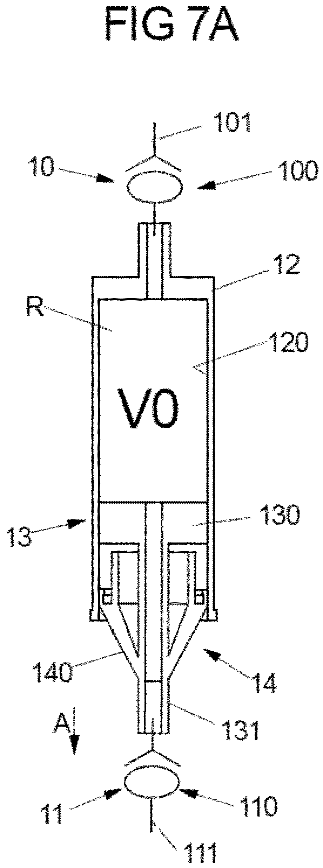

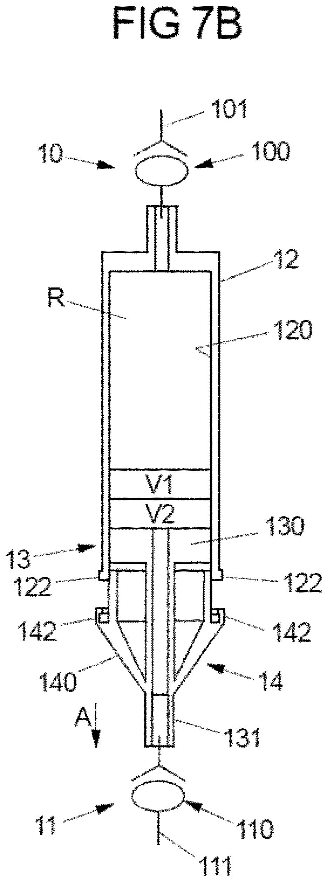

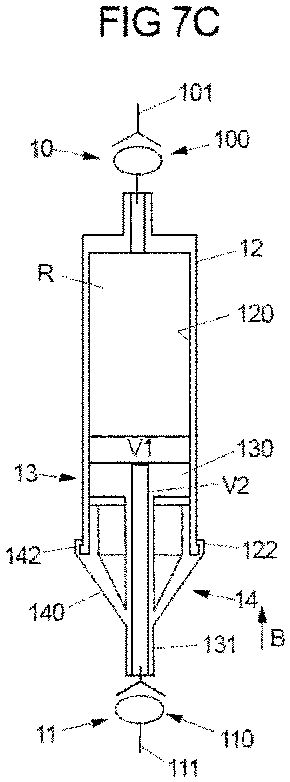

In another embodiment shown in Figs. 7A to 7C, a stopper member 14 in an initial position (Fig. 7A) is confined in the chamber body 12, wherein during a first actuation movement A the stopper member 14 is moved out of the chamber body 12 such that it radially is widened (Fig. 7B). During a second actuation movement B counter to the first actuation movement A the stopper member 14 is approached towards the chamber body 12 until the stopper member 14 with a stop element 140 interacts with the chamber body 12, hence blocking a further movement of the adjustment member 13 (Fig. 7C).

The stopper member 14 comprises a detent device 142 which engages with a detent device 122 of the chamber body 12 at an edge facing the stopper member 14, hence establishing a detent connection in the primed position according to Fig. 7C. The adjustment member 13 hence is held in the primed position according to Fig. 7C and cannot be easily moved out of the primed position, in particular in a direction for increasing the volume of the inner space R confined within the chamber body 12.

Functionally, the embodiment of Figs. 7A to 7C is identical to the embodiment of Figs. 4 and 5A to 5C, such that it also shall be referred to the explanations above.

By means of the drip chamber device 1 as proposed herein a priming is possible by two distinct actuation movements. Priming hence is made easy and comfortable for a user, wherein the priming procedure is fast.

The idea underlying the invention is not limited to the embodiment described above, but may be implemented in an entirely different fashion.

In the shown embodiment the outflow end is formed on the adjustment member. In another embodiment, the outflow end may be formed on the chamber body, such that a line section forming the outflow end is not moved together with the adjustment member during a priming procedure.

The stopper member may be formed by an elastic arrangement of a stop element, for example by an elastic detent element which is elastically pre-tensioned into a stop configuration, but may be brought into a release configuration and may be confined in the release configuration for example by interaction with the chamber body. Other designs of a stopper member then described herein however are conceivable.

### List of reference numerals

- 1: Drip chamber device
- 10: Inflow end
- 100: Valve device
- 101: Inflow line
- 11: Outflow end
- 110: Valve device
- 111: Outflow line
- 12: Chamber body
- 120: Wall
- 121: Opening
- 122: Detent device
- 123: Floor
- 13: Adjustment member
- 130: Closing section
- 131: Line section
- 14: Stopper member
- 140: Stop element
- 141: Pre-tensioning element
- 142: Detent device
- 2: Infusion line
- 20: Tubing
- 21: Control device
- 22: Connector
- 3: Fluid container
- A: First actuation movement
- B: Second actuation movement
- F: Fluid
- P: Patient
- R: Inner space
- V0, V1, V2: Volume

## Claims

1. A drip chamber device (1) for use on an infusion line (2) for administering a medical fluid (F) from a medical container (3) to a patient (P), comprising:
a chamber body (12) forming a wall (120) confining an inner space (R) of the chamber body (12),
an inflow end (10) for allowing the medical fluid (F) to flow into said inner space (R) of the chamber body (12) in a downstream direction, and
an outflow end (11) for allowing the medical fluid (F) to exit from said inner space (R) of the chamber body (12) in said downstream direction,
wherein an adjustment member (13) movable relative to said wall (120) of the chamber body (12), said adjustment member (13) comprising a closing section (130) which in a first position of the adjustment member (13) defines a first volume (V0) of the inner space (R) and is movable within said chamber body (12) to change the volume of the inner space (R),
**wherein** the adjustment member (13) comprises a line section (131) connected to the closing section (130) for conducting fluid, the line section (131) forming the outflow end (11) of the drip chamber device (1),
wherein the adjustment member (13) comprises a stopper member (14) configured to allow a first actuation movement (A) of the adjustment member (13) towards a second position in a first direction for increasing the volume of the inner space (R), but to stop a second actuation movement (B) of the adjustment member (13) from the second position in a second direction opposite to said first direction,
wherein said stopper member (14) is configured to stop said second actuation movement (B) after a decrease of the volume of the inner space (R) by a pre-defined volume (V2) with respect to the volume of the inner space (R) in said second position,
and **characterised in that** the decrease of the volume of the inner space volume due to the second actuation movement is less than the increase of the volume of the inner space due to the first actuation movement.

2. The drip chamber device (1) according to claim 1 or 2, **characterized in that** the closing section (130) is slidably movable along said wall (120) of the chamber body (12).

3. The drip chamber device (1) according to one of claims 1 to 3, **characterized in that** the inflow end (10) is arranged on the chamber body (12).

4. The drip chamber device (1) according to claim 5, **characterized in that** the stopper member (14) comprises a stop element (140) adjustable with respect to the closing section (130) of the adjust member (13) between a release configuration and a stop configuration, wherein the stop element (140) is configured to assume the release configuration at least in the beginning of the first actuation movement (A) of the adjustment member (13) and to assume the stop configuration in the second position of the adjustment member to stop said second actuation movement (B) of the adjustment member (13).

5. The drip chamber device (1) according to claim 6, **characterized in that** the stopper member (14) comprises a pre-tensioning element (141) for pre-tensioning the stop element (140) towards the stop configuration.

6. The drip chamber device (1) according to claim 6 or 7, **characterized in that** the stop element (140) in the first position of the adjustment member (13) is held in the release configuration by interaction with said chamber body (12).

7. The drip chamber device (1) according to one of the preceding claims, **characterized by** a first valve device (100) arranged at the inflow end (10) and/or a second valve device (110) arranged at the outflow end (11).

8. The drip chamber device (1) according to claim 9, **characterized in that** said first valve device (100) and/or said second valve device (110) is configured as a non-return valve.

9. The drip chamber device (1) according to claim 9 or 10, **characterized in that** said first valve device (100) and/or said second valve device (110) is configured to allow the medical fluid (F) to pass in said downstream direction only if a fluid pressure in the downstream direction is larger than a predefined threshold.

10. An infusion line set (2) comprising a tubing (20) for conducting a medical fluid (F) and a drip chamber device (1) according to one of the preceding claims connected to the tubing (20).

11. A method for priming a drip chamber device (1) according to one of claims 1 to 11, comprising:
moving the adjustment member (13) of the drip chamber device (1) relative to the chamber body (12) of the drip chamber device (1) from the first position towards a second position to increase the volume of the inner space (R) with respect to the volume of the inner space (R) in the first position, and
moving the adjustment member (13) of the drip chamber device (1) relative to the chamber body (12) of the drip chamber device (1) from the second position to decrease the volume of the inner space (R) with respect to the volume of the inner space (R) in the second position,
stopping a second actuating movement after a predefined distance by the stopper member (14).

## Patentansprüche

1. Tropfkammervorrichtung (1) zur Verwendung an einer Infusionsleitung (2) zur Verabreichung einer medizinischen Flüssigkeit (F) aus einem medizinischen Behälter (3) an einen Patienten (P), die Folgendes umfasst:
einen Kammerkörper (12), der eine Wand (120) bildet, die einen Innenraum (R) des Kammerkörpers (12) umschließt,
ein Einströmende (10), das der medizinischen Flüssigkeit (F) erlaubt, in einer Stromabwärtsrichtung in den Innenraum (R) des Kammerkörpers (12) einzuströmen, und
ein Ausströmende (11), das der medizinischen Flüssigkeit (F) erlaubt, in der Stromabwärtsrichtung aus dem Innenraum (R) des Kammerkörpers (12) auszutreten,
wobei ein Einstellelement (13) relativ zur Wand (120) des Kammerkörpers (12) beweglich, wobei das Einstellelement (13) einen Verschlussabschnitt (130) umfasst, der in einer ersten Position des Einstellelements (13) ein erstes Volumen (VO) des Innenraums (R) definiert und innerhalb des Kammerkörpers (12) beweglich ist, um das Volumen des Innenraums (R) zu verändern,
**wobei** das Einstellelement (13) einen Leitungsabschnitt (131) umfasst, der zum Leiten von Flüssigkeit mit dem Verschlussabschnitt (130) verbunden ist, wobei der Leitungsabschnitt (131) das Ausströmende (11) der Tropfkammervorrichtung (1) bildet,
wobei das Einstellelement (13) ein Anschlagelement (14) umfasst, das so konfiguriert ist, dass es eine erste Betätigungsbewegung (A) des Einstellelements (13) in Richtung einer zweiten Position in einer ersten Richtung zur Vergrößerung des Volumens des Innenraums (R) erlaubt, eine zweite Betätigungsbewegung (B) des Einstellelements (13) aus der zweiten Position in einer der ersten Richtung entgegengesetzten zweiten Richtung jedoch stoppt,
wobei das Anschlagelement (14) so konfiguriert ist, dass es die zweite Betätigungsbewegung (B) nach einer Abnahme des Volumens des Innenraums (R) um ein zuvor definiertes Volumen (V2) in Bezug auf das Volumen des Innenraums (R) in der zweiten Position stoppt,
und **dadurch gekennzeichnet, dass** die Abnahme des Volumens des Innenraumvolumens aufgrund der zweiten Betätigungsbewegung geringer ist als die Zunahme des Volumens des Innenraums aufgrund der ersten Betätigungsbewegung.

2. Tropfkammervorrichtung (1) nach Anspruch 1 oder 2, **dadurch gekennzeichnet, dass** der Verschlussabschnitt (130) entlang der Wand (120) des Kammerkörpers (12) verschiebbar beweglich ist.

3. Tropfkammervorrichtung (1) gemäß einem der Ansprüche 1 bis 3, **dadurch gekennzeichnet, dass** das Einströmende (10) am Kammerkörper (12) angeordnet ist.

4. Tropfkammervorrichtung (1) nach Anspruch 5, **dadurch gekennzeichnet, dass** das Anschlagelement (14) ein Anschlagelement (140) umfasst, das in Bezug auf den Verschlussabschnitt (130) des Einstellelements (13) zwischen einer Freigabekonfiguration und einer Anschlagkonfiguration verstellbar ist, wobei das Anschlagelement (140) so konfiguriert ist, dass es zumindest zu Beginn der ersten Betätigungsbewegung (A) des Einstellelements (13) die Freigabekonfiguration annimmt und in der zweiten Position des Einstellelements die Anschlagkonfiguration annimmt, um die zweite Betätigungsbewegung (B) des Einstellelements (13) zu stoppen.

5. Tropfkammervorrichtung (1) nach Anspruch 6, **dadurch gekennzeichnet, dass** das Anschlagelement (14) ein Vorspannelement (141) zum Vorspannen des Anschlagelements (140) in Richtung der Anschlagkonfiguration umfasst.

6. Tropfkammervorrichtung (1) nach Anspruch 6 oder 7, **dadurch gekennzeichnet, dass** das Anschlagelement (140) in der ersten Position des Einstellglieds (13) durch Zusammenwirken mit dem Kammerkörper (12) in der Freigabekonfiguration gehalten wird.

7. Tropfkammervorrichtung (1) nach einem der vorhergehenden Ansprüche, **gekennzeichnet durch** eine erste Ventileinrichtung (100), die am Einströmende (10) angeordnet ist, und/oder eine zweite Ventileinrichtung (110), die am Ausströmende (11) angeordnet ist.

8. Tropfkammervorrichtung (1) nach Anspruch 9, **dadurch gekennzeichnet, dass** die erste Ventilvorrichtung (100) und/oder die zweite Ventilvorrichtung (110) als Rückschlagventil konfiguriert ist/sind.

9. Tropfkammervorrichtung (1) gemäß Anspruch 9 oder 10, **dadurch gekennzeichnet, dass** die erste Ventilvorrichtung (100) und/oder die zweite Ventilvorrichtung (110) so konfiguriert ist/sind, dass sie der medizinischen Flüssigkeit (F) nur dann erlaubt/erlauben, in der Stromabwärtsrichtung zu passieren, wenn ein Flüssigkeitsdruck in der Stromabwärtsrichtung größer ist als ein zuvor definierter Schwellenwert.

10. Infusionsleitungsset (2), das einen Schlauch (20) zum Leiten einer medizinischen Flüssigkeit (F) und eine mit dem Schlauch (20) verbundene Tropfkammervorrichtung (1) nach einem der vorhergehenden Ansprüche umfasst.

11. Verfahren zum Vorbereiten einer Tropfkammervorrichtung (1) nach einem der Ansprüche 1 bis 11, das Folgendes umfasst:
Bewegen des Einstellelements (13) der Tropfkammervorrichtung (1) relativ zum Kammerkörper (12) der Tropfkammervorrichtung (1) aus der ersten Position in Richtung einer zweiten Position, um das Volumen des Innenraums (R) in Bezug auf das Volumen des Innenraums (R) in der ersten Position zu vergrößern, und
Bewegen des Einstellelements (13) der Tropfkammervorrichtung (1) relativ zum Kammerkörper (12) der Tropfkammervorrichtung (1) aus der zweiten Position, um das Volumen des Innenraums (R) in Bezug auf das Volumen des Innenraums (R) in der zweiten Position zu verringern,
Stoppen einer zweiten Betätigungsbewegung nach einer zuvor definierten Strecke durch das Anschlagelement (14).

## Revendications

1. Dispositif de chambre de gouttes (1) destiné à être utilisé sur une ligne de perfusion (2) pour l'administration d'un fluide médical (F) d'un contenant médical (3) à un patient (P), comprenant :
un corps de chambre (12) formant une paroi (120) confinant un espace interne (R) du corps de chambre (12),
une extrémité d'écoulement en entrée (10) destinée à permettre au fluide médical (F) de s'écouler dans ledit espace interne (R) du corps de chambre (12) dans une direction en aval, et
une extrémité d'écoulement en sortie (11) destinée à permettre au fluide médical (F) de sortir dudit espace interne (R) du corps de chambre (12) dans ladite direction aval,
dans lequel un organe de réglage (13) mobile relativement à ladite paroi (120) du corps de chambre (12), ledit organe de réglage (13) comprenant une section de fermeture (130) qui dans une première position de l'organe de réglage (13), définit un premier volume (VO) de l'espace interne (R) et est mobile au sein dudit corps de chambre (12) pour modifier le volume de l'espace interne (R),
**dans lequel** l'organe de réglage (13) comprend une section de ligne (131) reliée à la section de fermeture (130) destinée à conduire le fluide, la section de ligne (131) formant l'extrémité d'écoulement en sortie (11) du dispositif de chambre de gouttes (1),
dans lequel l'organe de réglage (13) comprend un organe bouchon (14) conçu pour permettre un premier mouvement d'actionnement (A) de l'organe de réglage (13) vers une seconde position dans une première direction pour l'augmentation du volume de l'espace interne (R), mais pour arrêter un second mouvement d'actionnement (B) de l'organe de réglage (13) de la seconde position dans une seconde direction opposée à ladite première direction,
dans lequel ledit organe bouchon (14) est conçu pour arrêter ledit second mouvement d'actionnement (B) après une diminution du volume de l'espace interne (R) d'un volume prédéfini (V2) par rapport au volume de l'espace interne (R) dans ladite seconde position,
et **caractérisé en ce que** la diminution du volume de l'espace interne due au second mouvement d'actionnement est inférieure à l'augmentation du volume de l'espace interne due au premier mouvement d'actionnement.

2. Dispositif de chambre de gouttes (1) selon la revendication 1 ou 2, **caractérisé en ce que** la section de fermeture (130) est mobile de façon coulissante le long de ladite paroi (120) du corps de chambre (12).

3. Dispositif de chambre de gouttes (1) selon l'une des revendications 1 à 3, **caractérisé en ce que** l'extrémité d'écoulement en entrée (10) est agencée sur le corps de chambre (12).

4. Dispositif de chambre de gouttes (1) selon la revendication 5, **caractérisé en ce que** l'organe bouchon (14) comprend un élément d'arrêt (140) réglable par rapport à la section de fermeture (130) de l'organe de réglage (13) entre une configuration de libération et une configuration d'arrêt, dans lequel l'élément d'arrêt (140) est conçu pour prendre la configuration de libération au moins au début du premier mouvement d'actionnement (A) de l'organe de réglage (13) et pour prendre la configuration d'arrêt dans la seconde position de l'organe de réglage pour arrêter ledit second mouvement d'actionnement (B) de l'organe de réglage (13).

5. Dispositif de chambre de gouttes (1) selon la revendication 6, **caractérisé en** ceque l'organe bouchon (14) comprend un élément de pré-tension (141) destiné à pré-tendre l'élément d'arrêt (140) vers la configuration d'arrêt.

6. Dispositif de chambre de gouttes (1) selon la revendication 6 ou 7, **caractérisé en ce**que l'élément d'arrêt (140) dans la première position de l'organe de réglage (13) est maintenu dans la configuration de libération par interaction avec ledit corps de chambre (12).

7. Dispositif de chambre de gouttes (1) selon l'une des revendications précédentes, **caractérisé par** un premier dispositif de vanne (100) disposé au niveau de l'extrémité d'écoulement en entrée (10) et/ou un second dispositif de vanne (110) disposé au niveau de l'extrémité d'écoulement en sortie (11).

8. Dispositif de chambre de gouttes (1) selon la revendication 9, **caractérisé en ce que** ledit premier dispositif de vanne (100) et/ou ledit second dispositif de vanne (110) est conçu comme un clapet anti-retour.

9. Dispositif de chambre de gouttes (1) selon la revendication 9 ou 10, **caractérisé en ce que** ledit premier dispositif de vanne (100) et/ou ledit second dispositif de vanne (110) est conçu pour permettre au fluide médical (F) de passer dans ladite direction aval seulement si une pression de fluide dans la direction aval est supérieure à un seuil prédéfini.

10. Ensemble de lignes de perfusion (2) comprenant une tubulure (20) destinée à conduire un fluide médical (F) et un dispositif de chambre de gouttes (1) selon l'une des revendications précédentes raccordé à la tubulure (20).

11. Procédé d'amorçage d'un dispositif de chambre de gouttes (1) selon l'une des revendications 1 à 11, comprenant :
le mouvement de l'organe de réglage (13) du dispositif de chambre de gouttes (1) relativement au corps de chambre (12) du dispositif de chambre de gouttes (1) depuis la première position vers une seconde position pour augmenter le volume de l'espace interne (R) par rapport au volume de l'espace interne (R) dans la première position, et
le mouvement de l'organe de réglage (13) du dispositif de chambre de gouttes (1) relativement au corps de chambre (12) du dispositif de chambre de gouttes (1) depuis la seconde position pour diminuer le volume de l'espace interne (R) par rapport au volume de l'espace interne (R) dans la seconde position,
l'arrêt d'un second mouvement d'actionnement après une distance prédéfinie par l'organe de bouchon (14).
